# EUROPEAN PATENT APPLICATION

(11) **EP 1 298 201 A1**
(43) Date of publication of application: **02.04.2003**
(21) Application number: 01123139.6
(22) Date of filing: 27.09.2001
(51) Int. Cl.: C12N 5/06, A61P 5/48, A61K 35/39, C12Q 1/02

(54) **Process for the production of cells exhibiting an islet-beta-cell-like state**

(71) Applicant: Cardion AG, 40699 Erkrath (DE)
(72) Inventor: Escoms, Bernat Soria, 03540 Playa de San Juan, Alicante (ES); Quinto, Trinidad Le n, 03540 Playa de San Juan, Alicante (ES); Jones, Jonathan, 03001 Alicante (ES); Haym, Anouchka Skoudy, 08002 Barcelona (ES); Arribas, Francisco Real, 08328 Alella, Barcelona (ES)

(57) **Abstract**

The invention relates to a process for the in vitro-directed differentiation of omnipotent or pluripotent cells into islet precursors, wherein at least one compound selected from the group consisting of embryonic pancreases and parts thereof, sonic hedgehog inhibitors, especially cyclopamin, anti-Sonic hedgehog or basic fibroblast growth factor, nicotinamide, LY294002, sodium tungstate, laminin-1 or another extracellular matrix protein and all-trans retinoic acid is used for the differentiation.

## Description

The present invention relates to a process for the differentiation of pluripotent cells into insulin-producing cells, preferably islet precursor cells, as a promising prerequisite for the treatment of type 1 diabetes.

The maintenance of glucose concentration in blood within the appropriate range is controlled mainly by the secretion of pancreatic islet hormones, principally insulin from the beta-cells of the islet of Langerhans. Absence or diminution of insulin release results in types 1 and 2 diabetes, respectively. Diabetes mellitus (DM) is a heterogeneous metabolic disorder affecting 2-5 % of the adult population in developed countries. DM can be classified broadly into two groups: the insulin-dependent type (IDDM or type 1) and the non-insulin-dependent type (NIDDM or type 2). Treatment of type-1 DM is still based on self-injection of insulin 3-4 times daily. However, this does not prevent the long-term appearance of microvascular complications including diabetic retinopathy, nephropathy or neuropathy (The Diabetes Control and Complications Trial (1993) N. Eng. J. Med. 329: 6510-6520). It has been shown that intensive insulin therapy can delay the onset and diminish the progression of microvascular complications in type-1 diabetes. However, this kind of treatment needs motivated patients and does not liberate them from insulin dependence.

By restoring β-cell function with new therapeutic strategies, including transplantation of exogenous β-cells (islet allotransplantation and xenotransplantation, implanting β-cell surrogates, etc.) patients can be free from insulin therapy. On the other hand, the use of gene therapy for the treatment of diabetes still needs crucial improvements (Efrat S (1998) Diabetologia 41: 1401-1409). By using specific promoters, the insulin gene has been successfully expressed in a broad variety of tissues, including skeletal muscle, exocrine pancreas, and salivary glands (Gros L. Montoliu L, Riu E, Lebrigand L, Bosch F (11998) Human Gene Ther 8: 2249-2259; Goldfine ID, German MD, Tseng HC, Wang J, Bolaffi JL, Chen JW,Olson DC, Rothman SS (1997) Nat. Biotechnol. 15: 1378-1382). The use of mutated proinsulin at the level of the cleavage sites allows correct processing of proinsulin to mature insulin by furins (Kaufman JE, Irminger JC, Mungall J, Halban PA (1997) Diabetes 46: 978-982). However the main obstacle of ectopic insulin expression is to mimic the modulation of the β-cell secretory pattern in non-β-cells.

The difficulty encountered in reconstructing the β-cell function ectopically makes exogenous β-cells an alternative choice for restoring β-cell mass or β-cell function in either type 1 or type 2 diabetes. Allotransplantation or xenotransplantation still imposes important problems: scarcity of material, technical difficulty, high costs, risk of infection by endogenous animal retroviruses, and immunological rejection, which lead to the inherent problems of macroencapsulation and microencapsulation strategies.

In islet transplantation two or more pancreases are needed per diabetic patient to reach insulin independence (Shapiro AMJ, Lakey JRT, Ryan EA, Korbutt GS, Toth E, Warnock GL, Kneteman NM, Rajotte RV (2000) N. Eng. J. Med. 343: 230-238). An estimation of the organ procurement versus organ needs to treat type 1 diabetic people demonstrates that even in countries like Spain with 30-40 donors per million people per year, there is a need for 4-fold more donors. Engineered pancreatic islet cells could be a potential solution for the scarcity problem. The use of tumoral and transformed insulin-producing cell lines has been proposed as one of the alternatives. Clones displaying the most appropiate patterns of insulin secretion can be selected and amplified. Furthermore, cell lines may be engineered to be immunoprotected. However, since these cells display uncontrolled proliferation and unstable phenotype their use in cell therapy is questionable.

Engineered pancreatic B-cells may fulfill the actual needs to finally find a cure for type 1 autoimmune diabetes. Recently it has been shown that mouse embryonic stem cells may be driven to form insulin-containing cells in vitro [Soria B, Roche E, Berna G, Leon-Quinto T, Reig JA, Martín F (2000) Diabetes 49: 157-162]. Subsequent cell lineage selection with a cell trapping system based in the expression of a reporter gene [Soria B, Roche E, Berna G, Leon-Quinto T, Reig JA, Martin F (2000) Diabetes 49: 157-162] allowed to obtain a cell line that normalizes hyperglycaemia and restores body weight when transplanted into the spleen of streptozotocin-diabetic mice.

A disadvantage of this method is, that some islet markers, such as islet hormones (insulin, glucagon, somatostatin, PP), B-cell enzymes, transporters and ionic channel elements (glucokinase, Glut-2, SUR-1) or transcription factors (PDX-1) may be expressed either in undifferentiated embryonic stem cells or during embryoid body formation. Further some of these factors are not indicative of islet cell terminal differentiation. For example, Beta2/NeuroD and Neurogenin3 expression is not restricted to endocrine pancreas. Further, early expressed insulin as another example is also a growth factor for retinal development and it has been shown that insulin is secreted by embryonic tissues during the prepancreatic stage of mouse development (De Pablo F, de la Rosa EJ (1995) Trends in Neuroscience 18: 143-150; Hernández-Sánchez C, Lopez-Carranza A, Alarcón C, de la Rosa EJ, de Pable F (1995) Proc. Natl. Acad. Sci. USA 92: 9834-9838; Spaventi R, Antica M, Pavelic K (1990) Development 108: 491-495 ]. Consequently, cell trapping systems may result in a high proportion of immature B-cells or in cells which are not committed to be islet cells.

Beyond that the proportion of cells which are positive for islet markers is very small (less than 1 %), then forcing to use more aggressive cell selection procedures or making more difficult cell lineage selection.

Another point to take into consideration is the big variability both between different cell lines as well as within a particular cell line with regard to the characteristics of the cells which can be isolated by such a cell trapping system. The cells by no way can be considered to be a homogeneous population. This is reflecting an uncontrolled behaviour of the cells which is especially undesired, if the cells are going to be used for therapeutic cloning. Any attempt to overcome this problem and to provide a process for the controlled production of insulin-containing cells has not been successful yet (Schuldiner M, Yanuka O, Iskovitz-Eldor J, Melton D, Benvenisty N (2000) Proc. Natl. Acad. Sci USA 97: 11307-11312).

It is therefore object of the present invention to provide a new process for the production of pancreatic beta-cells or pancreatic beta-cell-like cells, for which there is an urgent need with respect to the treatment of of the diabetes type 1.

Object of the present invention is in particular to provide a process, especially an in vitro process, for the controlled production of pancreatic beta-cells or pancreatic beta cell-like cells, especially of such with a precisely regulated insulin release, starting from cells with a lower degree of differentiation, especially totipotent or pluripotent cells.

The object is achieved by providing a process, in which cells which according to their state of differentiation are able to differentiate to islet beta cells or islet beta cell-like cells, in the following called "omnipotent or puripotent cells", are forced to differentiate to form such islet-beta cells or islet-beta-cell-like cells by the addition of pancreases or parts thereof and/or other compounds selected from the group consisting of anti-Sonic Hedgehog (anti-Shh), sonic hedgehog inhibitors, especially cyclopamine, nicotinamide, LY294002, basic Fibroblast Growth Factor, especially bFGF or FGF2, sodium tungstate, retinoic acid, laminin-1 and other extracellular matrix proteins, wherein the process preferably is an in vitro-process and wherein the process preferably allows the controlled production of said islet beta cells or islet beta cell-like cells.

Each factor used for the differentiation is selected according to its role in pancreatic development. Thus, pancreas development is promoted by sonic hedgehog inhibitors like cyclopamine (Incardona, JP, Gaffield, W, Kapur, RP, Roelink, H (1998) Development 125: 3553-3562; Kim SK, Melton DA (1998) Proc Natl Acad Sci USA 95: 13036-13041), anti-Sonic hedgehog (Marti E, Bumcrot DA, Takada R, McMahon AP (1995) Nature 375: 279-280) or basic fibroblast growth factor (bFGF or FG2) (Hebrok M, Kim SK, Melton DA (1998) Genes Dev 12: 1705-1713). Nicotinamide is a powerful inducer of endocrine differentiation in cultured human fetal pancreatic cells (Otonkoski, T, Beattie, GM, Mally, Ml, Ricordi, C, Hayek, A (1993) J. Clin. Invest. 92: 1459-1466). Laminin-1 promotes differentiation of fetal mouse pancreatic B-cells (Jiang, F-X, Cram, DS, DeAizpurua, HJ, Harrison, LC (1999) Diabetes 48: 722-730). LY294002 blockades P13K activity in human fetal undifferentiated cells inducing endocrine differentiation (Ptasznik, A, Beattie, GM, Mally, Ml, Cirulli, V, Lopez, A, Hayek, A (1997) J. Cell Biol. 137: 1127-1136). Retinoic acid induces Pax6 expression (Gajovic S, St-Onge L, Yokota Y, Gruss P (1997) Differentiation 62: 187-192), a transcription factor present during development of the central nervous system and during development of islet cells (St-Onge L, Sosa-Pineda B, Chowyhur K, Mansouri A, Gruss P (1997) Nature 387: 399-402). On the other hand, retinoic acid stimulated glucokinase activity in both fetal islets and differentiated adult islets in culture (Cabrera-Valladares G, German MS, Matchinsky FM, Wang J, Fernández-Mejía C (1999) Endocrinology 140: 3091-3096). Oral administration of sodium-tungstate to streptozotocin-treated rats caused a partial recovery in the insulin content and in preproinsulin mRNA levels in their islets (Barbera A, Fernandez-Alvarez J, Truc A, Gomis R, Guinovart JJ (1997) Diabetologia 40: 143-149). Low glucose partially recovered the depletion in insulin stores and the lowered binding activity of Pdx-1 (pancreatic and duodenal homeobox-factor-1) induced by culture of human islets in high glucose (Marshak S,Leibowitz G, Bertuzzi F, Socci C, Kaiser N, Gross DJ, Cerasi E, Melloul D (1999) Diabetes 48: 1230-1236). Embryonic pancreases were selected from e14.5, e15.5 and e17.5 because formation of B-cells during development only begins in significant numbers at e13.5 (Wells JM, Melton DA (1999) Annual Review of Developmental Biology 15; 393-410).

With "cells able to differentiate to islet-beta cells or islet beta cell-like cells" and especially with "omnipotent or pluripotent cells" are meant for example stem cells, especially embryonic stem cells or adult stem cells or progenitors thereof, but also any other cells which are able to differentiate to islet-beta-cell-like cells, like for example cells from the pancreatic bud, cells from the foregut, endodermal or endocrine cells.

By "stem cell" is meant in general a cell which can undergo self-renewing cell division to give rise to phenotypically and genotypically identical daughters and ultimately can differentiate into at least one final cell type.

Stem cells also include embryonic germ cells, embryonic carcinoma cells, fetal pancreatic stem cells, pancreatic duct cells and nestin-positive cells (Hunzinger E, Stein M (2000) Biochem. Biophys. Res. Commun. 271: 116-119; Lumelsky N, Blondel O, Laeng P, Velasco I, Ravin R, McKay R (2001) Science 292: 1389-1394; Zulewski H, Abraham EJ, Gerlach MJ, Daniel PB, Moritz W, Müller W, Vallejo M, Thomas MK, Habener JF (2000) Diabetes 50: 521-533).

By "adult stem cells or progenitors thereof' are meant cells that can be derived from any source of adult tissue or organ and that can replicate as undifferentiated cells and have the potential to differentiate into at least one, preferably multiple cell lineages, about all are meant herewith pluripotent cells from the adult, such as for example bone marrow stroma cells (Woodbury D, Schwarz EJ, Prockop DJ, Black IB (2000) J. Neurosc. Res. 61: 364-370), neural stem cells (Clarke DL, Johansson CB, Wilbertz J, Veress B, Nilsson E, Karlström H, Lendahl V, Frisen J (2000) Science 288: 1660-1663) and intestinal epithelia stem cells (Martin F, Berná G, Enseñat-Wasser R, Reig JA, Soria B, unpublished).

With "islet beta cell-like cells" are especially meant cells like beta-cell-precursors or any other cells which exhibit production and/or content, especially elevated production and/or content, of at least one islet marker, especially of insulin and/or of a transcription factor like Pdx-1, Nkx6.1, Neurogenin3 and/or Beta2/NeuroD, preferably of insulin and Pdx-1 simultaneously. In a special embodiment with "islet beta cell-like cells" are also meant beta-cell precursors, endocrine or endodermal cells.

In a preferred embodiment embryonal stem (ES) cells are used in the differentiation process. These cells can be derived from the inner cell mass of the blastocyst, or can be derived from the primordial germ cells from a post-implantation embryo (embryonal germ cells or EG cells). ES and EG cells have been derived from mice, and more recently also from non-human primates and humans (Evans MJ, Kaufman Nature. 1981 Jul 9;292(5819):154-6.; Matsui Y, Toksoz D, Nishikawa S, Nishikawa S, Williams D, Zsebo K, Hogan BL. Nature. 1991 Oct 24;353(6346):750-2.Thomson JA, Kalishman J, Golos TG, Durning M, Harris CP, Becker RA, Hearn JP.Proc Natl Acad Sci U S A. 1995 Aug 15;92(17):7844-8.; Thomson JA, Itskovitz-Eldor J, Shapiro SS, Waknitz MA, Swiergiel JJ, Marshall VS, Jones JM. Science. 1998 Nov 6;282(5391):1145-7; Shamblott MJ, Axelman J, Wang S, Bugg EM, Littlefield JW, Donovan PJ, Blumenthal PD, Huggins GR, Gearhart JD. Proc Natl Acad Sci U S A. 1998 Nov 10;95(23):13726-31.).

In an other preferred embodiment stem cells isolated from regenerative tissues are used in the differentiation process. Stem cells have been identified in most organs and tissues. The best characterized is the hematopoietic stem cell. The hematopoietic stem cell may give rise to any of the different types of terminally differentiated blood cells. This is a mesoderm derived cell that has been purified based on cell surface markers and functional characteristics (Hill B, Rozler E, Travis M, Chen S, Zannetino A, Simmons P, Galy A, Chen B, Hoffman R. Exp Hematol. 1996 Jul;24(8):936-43.). Also well characterized is the neural stem cell (Flax JD, Aurora S, Yang C, Simonin C, Wills AM, Billinghurst LL, Jendoubi M, Sidman RL, Wolfe JH, Kim SU, Snyder EY. Nat Biotechnol. 1998 Nov;16(11):1033-9.; D. L. Clarke et al., Science 288, 1666 (2000)) and a number of mesenchymal stem cells derived from multiple sources (Bruder SP, Jaiswal N, Haynesworth SE. J Cell Biochem. 1997 Feb;64(2):278-94.; Yoo JU, Barthel TS, Nishimura K, Solchaga L, Caplan Al, Goldberg VM, Johnstone B. J Bone Joint Surg Am. 1998 Dec;80(12):1745-57.; Makino S, Fukuda K, Miyoshi S, Konishi F, Kodama H, Pan J, Sano M, Takahashi T, Hori S, Abe H, Hata J, Umezawa A, Ogawa S. J Clin Invest. 1999 Mar;103(5):697-705; Pittenger MF, Mackay AM, Beck SC, Jaiswal RK, Douglas R, Mosca JD, Moorman MA, Simonetti DW, Craig S, Marshak DR. Science. 1999 Apr 2;284(5411):143-7.)

Cells from regenerative tissue for example may be isolated by a process comprising the following steps (a) to (c) and optionally the steps (d) and (e):
a) isolating a population derived from regenerative tissue containing somatic adult stem cells;
b) plating and culturing the population according to (a) on mitotically inactivated fibroblast feeder layer, preferably human;
c) selecting and replating colonies which have a pluripotent morphology;
d) expanding the cells of the selected colonies over several passages, for example 2-15 passages, preferably 10 - 15 passages;
e) causing differentiation by deleting fibroblast feeder layer and culturing at high density in suspension.

The cells used in the differentiation process exhibit a lower degree of differentiation than the resulting islet beta cells or islet beta cell-like cells.

The cells used in the differentiation process are preferably cells from mammals, for example such from mouse, human, cattle, sheep, rabbit, rat, pig or goat.

In a preferred embodiment at least 10 %, preferably at least 20 %, of the cells which were subjected to the differentiation process finally have differentiated to islet beta cells or islet beta cell-like cells and/or show an elevated and/or detectable concentration and/or content of at least one of the said islet markers. In a very preferred embodiment at least 8 % of the cells, preferably at least 15 % of the cells finally show an elevated and/or detectable amount of at least two of the said islet markers, wherein the two islet markers about all are insulin and pdx-1.

In a special embodiment the embryonic stem cell line D3 from mice is used. Further stem cell lines that can be used are for example R1 ( provided by J. Rossant, Mount Sinai Hospital, Toronto, Ontario, Canada), CGR8 ( Nichols J, Zevnik B, Anastassiadis K, Niwa H, Klebe-Nebenius D, Chambers I, Scholer H, Smith A (1998) Cell 95: 379-391) or ENS ( Gauthier K, Chassande O, Plateroti M, Roux JP, Legrand C, Pain B, Rousset B, Weiss R, Trouillas J, Samarut J (1999) EMBO J 18: 623-631).

For cultivation of the pluripotent cells the methods known to the person skilled in the art can be used. Cultivation of D3 cells for example can be carried out in high glucose Dulbecco's Modified Eagles Medium, DMEM (GIBCO/DRL, Grand Island, New York)) supplemented with heat-activated fetal bovine serum (FBS) (preferentially 15 %), non essential amino acids (preferentially 1 %), 0,1 mM 2-mercaptoethanol, 1 mM sodium pyruvate, penicillin/streptomycin (100 IU/0.1 mg/ml, respectively) and 2 mM glutamine as glutamax (Gibco). To maintain the pluripotent undifferentiated state Leukemia inhibitory factor (LIF) (1000 U/ml) or any other substance inhibiting cell differentiation can be added.

In vitro induction of cell differentiation and by that formation of the embryoid body is achieved by lacking the differentiation inhibitor. For this purpose the pluripotent cells for example can be transferred to a bacterial petri dish. For example between 1x10⁵ and 1x10⁸, preferentially about 5x10⁶ embryonic cells, can be transferred to a 100-mm bacterial petri dish, or accordingly more or less cells are transferred to a bigger or smaller petri dish to achieve the same cell density. Cells are cultured in serum free medium or fetal calf serum can be added to amount to a concentration of about 10 %. In a preferred embodiment of the invention a lower nutrient concentration, especially a lower concentration of fetal calf serum, is used, about all during the formation of the embryoid body, to increase the effect of the compounds tested. Therefore preferably a concentration between 0 and 6 %, especially between 2 and 5 %, preferably a concentration of about 3 % fetal calf serum is used.

After preferably between 4 and 12 days, especially after about 7 days in the suspension culture, the formed embryoid body can be plated on a culture plate, preferably on a tissue culture plate, especially on a gelatin-coated culture plate or on a culture plate containing similar material. For example a 100 mm tissue culture plate can be used for the said cells of the suspension culture of the 100 mm petri dish. Plating occurs preferably after disgregating the embryoid body, which can be done for example mechanically.

On the plates incubation is taking place for example between 4 and 20 days, preferably between 6 and 15 days, about all for about 7 or for about 14 days.

For the differentiation of the cells preferably exogenous factors and/or pancreases are used. In a prefered embodiment the factors and/or pancreases are added to the culture right when the factor inhibiting cell differentiation, like LIF (PACISA-GIRALT, Madrid, TO1-ESG1106) [Smith AG, Jeath JK, Donaldson DD, Wong GG, Moreau J, Stahl M, Rogers D (1988) Nature 336: 688-690; Williams RL, Hilton DJ, Pease S, Willson TA, Stewart CL, Gearing DP, Wagner EF, Metcalf D, Nicola NA, Gough NM (1988) Nature 336: 684-687], or any other factor or condition maintaining pluripotentiality, is leaft out from the medium. That means the factors and/or pancreases are added preferably during the formation of the embryoid body, about all from the beginning of the formation of the embryoid body, so that differentiation of the cells is promoted through the desired pathways and undesirable spontaneous cell differentiation is avoided.

To induce the desired cell differentiation the medium is supplemented with a single factor or pancreas or with any combination of the following factors and pancreases, wherein the factors preferably are used in the said concentrations: nicotinamide (SIGMA, N-3376) [Otonkoski, T, Beattie, GM, Mally, Ml, Ricordi, C, Hayek, A (1993) J. Clin. Invest. 92: 1459-1466] in a concentration between 2 and 50 mM, especially in a concentration between 5 and 20 mM, about all in a concentration of about 10 mM, LY294002 (CALBIOCHEM, 440202) [Ptasznik, A, Beattie, GM, Mally, Ml, Cirulli, V, Lopez, A, Hayek, A (1997) J. Cell Biol. 137: 1127-1136.] in a concentration between 2 and 100 µM, especially in a concentration of about 10, about 15 or about 20 µM, sodium tungstate ( SIGMA T2629) [Barbera A, Fernandez-Alvarez J, Truc A, Gomis R, Guinovart JJ (1997) Diabetologia 40: 143-149 ] in a concentration between 50 nM and 500 µM, especially in a concentration between 100 nM and 100 µM, about all in a concentration of about 1 or 10 µM, laminin-1 (GIBCO 12163-010) [Jiang, F-X, Cram, DS, DeAizpurua, HJ, Harrison, LC (1999) Diabetes 48: 722-730] in a concentration between 8 and 200 µg/ml, especially in a concentration between 25 and 70 µg/ml, about all in a concentration of about 40 µg/ml, cyclopamine (provided by W. Gaffield, US Dept of Agriculture, Albany, CA) [Incardona, JP, Gaffield, W, Kapur, RP, Roelink, H (1998) Development 125: 3553-3562; Kim, SK, Melton, DA (1998) Proc. Natl. Acad. Sci. USA 95: 13036-13051 ] in a concentration between 20 nM and 100 µM, about all in a concentration of about 0,1 or about 20 µM, anti-Sonic hedgehog (Developmental Studies Hybridoma Bank, The University of Iowa, Iowa City, IA) [Marti E, Bumcrot DA, Takada R, McMahon AP (1995) Nature 375: 279-280; Hebrok M, Kim SK, Melton DA (1998) Genes Dev 12: 1705-1713 ] in a concentration between 1 and 50 µg/ml, especially in a concentration between 2 and 20 µg/ml, about all in a concentration between 5 and 10 µg/ml, all-trans retinoic acid (SIGMA R2625) [Gajovic S, St-Onge L, Yokoba Y, Gruss P (1997) Differentiation 62: 187-192] in a concentration between 0,2 and 5 µM, especially in a concentration of about 1 µM, basic fibroblast growth factor, especially bFGF or FGF2, in a concentration between 1 and 100 ngr/ml, about all in a concentration of about 10 ngr/ml. The glucose (PANREAC 141341) concentration was kept preferably between 1 and 25 mM, especially between 3 and 10 mM, about all a glucose concentration of about 5 mM was used in the tests, especially during the formation of the embryoid body.

The pancreases which can be used according to the invention to induce cell differentiation preferably are embryonic pancreases or parts thereof, especially embryonic pancreatic buds and pancreatic rudiments. In a special embodiment of the invention mouse pancreases between embryonic day 12 and 20, preferably between embryonic day 14 and 18, about all pancreasses of about embryonic day 14.5, 15.5 or 17.5 (e14.5, e15.5 or e17.5) were used. Since interspecific effects are observed, for example pancreases from mouse, chicken, human, goat, rat, pig, rabbit or cattle may be used to promote differentiation on stem cells of different origins.

The pancreases can for example be isolated by microdissection and liberation in Hank's balanced solution (GIBCO). After washing in Hank's balanced solution for several times the pancreases can be transferred to the petri dishes containing the pluripotent cells. After incubation the pancreasses can be seperated from the pluripotent cells by filters (Millipore, Bedford, MA, USA). In a special embodiment embryos from time-mated pregnant OF1 mice are used for the isolation of the pancreases.

Treatment of the cells with the differentiation factors and/or embryonic pancreasses can be done once, several times, often or continuosly, periodically or not periodically. In a preferred embodiment treatment is occurring at least during the formation of the embryoid body.

It can be added a singular of the said compounds and pancreases or any combination thereof. The different compounds and pancreases can be added simultaneouly, successive or incubation of the pluripotent cells with the different compounds and pancreasses can occur in overlapping time-intervals.

In a preferred embodiment of the invention addition of anti-sonic hedgehog, laminin-1 and/or of the embryonic pankreasses occurs only during the suspension phase, where the formation of the embryoid body is taking place, while nicotinamide, LY294002, sodium tungstate, retinoic acid and/or cyclopamine are added continuosly at periodic intervals, also after the embryoid body has been plated on the culture plates.

In a special embodiment of the invention a cell-trapping system is used to identify and select cells which show beta-cell-like characteristics and vice versa to eliminate cells which do not exhibit the desired behaviour. For example cell-trapping systems can be used as described in Soria B, Roche E, Berna G, Leon-Quinto T, Reig JA, Martin F (2000) Diabetes 49: 157-162; Soria B, Skoudy A, Martin F (2001) Diabetologia 44: 407-415; Berná G, León-Quinto T, Enseñat-Wasser R, Montanya E, Martín F, Soria B (2001) Biomed. Pharmacother. 55: 206-212.

For this purpose the pluripotent cells for example are first transfected with a DNA that comprises at least one characteristic promoter of the differentiation process of beta-cells, what means a promoter which is switched on during the beta-cell formation or during the formation of a beta-cell precursor starting from pluripotent cells. This promoter for example may be the promoter of the insulin gene.

Under control of this promoter is preferably at least one gene, which is coding for a peptide that can provide resistance against at least one otherwise toxic substance, especially against an antibiotic. An example of such a gene is the β-geo gene (P.Soriano, Fred Hutchinson Cancer Research Center, Seattle, Washington) which if expressed provides resistance towards G-418, so that cells which contain the expression product β-geo can survive in a medium containing G-418. By that way for example insuline producing cells or cells which show some other characteristics of beta-cells or beta-cell precursors can be identified and selected using G-418.

Preferably at least one other gene might be additionally localised on the DNA used for transfection to enable the discrimination between transfected cells and cells which have not been transfected. For this purpos for example pGK/Hygro can be used, which is enabling the cells containing its expression product to grow on hygromycin, while cells without that gene can not. Furthermore, a safety system may be incorporated comprising the ligation of the Herpes thymidine kinase gene after PGK-Hygro, thus allowing cell suppression by ganciclovir administration to the transplanted patient.

In another preferred embodiment the cells obtained by the differentiation process are used for a therapeutical purpose, especially for transplantation, about all for spleen transplantation. The transplantation for example may be carried out by lumbar incision, followed by injection of the cells into the spleen. Cells may also be transplanted into the liver, the renal capsula, under the skin and other places of the body.

Subject matter of the invention therefore further is a drug product comprising a differentiated cell according to the invention, especially a pancreatic beta-cell or pancreatic beta-cell-precursor, either in naked form or in an embodiment, where the cells are formulated together with pharmaceutically acceptable additives and/or adjuvants.

Subject matter of the invention therefore is further the therapeutic use of such a drug product, especially transplantation, about all liver, spleen, renal capsula, subcutaneous transplantation and transplantation into other sites.

Subject matter of the invention therefore further is a process for the preparation of a drug product for the treatment of diabetes, where at least one differentiated cell according to the invention is formulated, if appropriate, together with a pharmaceutically acceptable additive and/or adjuvant, preferably in a form which is effective in cell and/or gene therapy.

Subject matter is therefore further a diagnostic agent comprising a differentiated cell according to the invention, especially a beta-cell or beta-cell-like cell, either in naked form or in an embodiment, where the at least one cell is formulated with suitable additives and/or adjuvants.

Subject matter is therefore further a differentiated cell according to the invention, especially a beta-cell or beta-cell-like cell, either in naked form or in an embodiment, used for screening of drugs, toxics and other products.

Subject matter of the invention is therfore also the preparation of a diagnostic agent for diagnosing diabetes, where at least one differentiated cell of the invention, especially a beta-cell or beta-cell-like cell, is combined with suitable additives and/or adjuvants or a pharmaceutically acceptable carrier.

### Examples

### Cell culture

### Undifferentiated cell culture

Cells of the embryonic stem cell line D3 (American Type Culture Collection, Rockville, MD) were cultivated in high glucose DMEM (GIBCO 32430-027) supplemented with 15% heat-inactivated fetal bovine serum (FBS), 1% non essential aminoacids, 0.1 mM 2-mercaptoethanol, 1 mM sodium pyruvate, penicillin/streptomycin (100 IU/0.1 mg/ml, respectively) and 2 mM glutamine as glutamax (GIBCO). Leukemia inhibitory factor (1000 U/ml) was added to maintain the pluripotent undifferentiated state.

### In vitro differentiation

To induce differentiation, about 5x10⁶ embryonic stem (ES) cells were transferred to a 100-mm bacterial petri dish to allow formation of embryoid bodies. After 7 days in suspension culture, the resulting embryoid bodies were mechanically disgregated and then plated onto a gelatin-coated 100-mm tissue culture plate during 7 additional days. The cells were cultured in complete medium without LIF and supplemented with 3% FBS during the suspension culture and with 10% during the subsequent plating and culture from 7 to 14 days.

Differentiation was induced by exogenous factors and embryonic pancreases microdissected. The factors used were: nicotinamide, 10 mM; LY294002, 10, 15 and 20 µM; sodium tungstate, 1, 10 and 100 µM; glucose 5 mM; laminin-1, 40 µgr/ml cyclopamine 0.1 and 20 µM ; anti-Sonic hedgehog 5 and 10 µgr/ml; all-trans retinoic acid, 1µM; basic fibroblast growth factor 5 and 10 ngr/ml. Embryos from time-mated pregnant OF1 mice (e14.5, e15.5 or e17.5) were liberated in Hank's balanced salt solution (GIBCO 14170-088). Whole pancreases were collected by microdissection, washed in Hank's several times and transferred to petri dishes. Whole pancreases from e14.5, e15.5 or e17.5 were used as differentiation agents and separated from ES cells by filters (Millipore, Bedford, MA). The cells were treated with such factors and embryonic pancreases during defined intervals of time. Thus, ES cells were continuosly exposed to nicotinamide, LY294002, sodium tungstate, low glucose, retinoic acid or cyclopamine whereas anti-sonic hedgehog, laminin-1 and the embryonic pancreases were only present in the culture medium during the 7 days of suspension culture.

### Semiquantitative reverse transcription-polymerase chain reaction

The differentiation induced by the agents was examined by determining the expression of pancreatic genes by using RT-PCR. RNA was isolated from various samples at distinct stages of maturation. Total RNA was isolated after lysing the cells in a guanidinium thiocyanate/phenol buffer. cDNA was generated and transcripts were amplified by specific primers to these genes. DNasel-treated total RNA (1µg) was reverse-transcripted and cDNA were amplified using the Superscript one step RT-PCR kit (GIBCO). The PCR product was finally subjected to agarose gel electrophoresis to visualize the effect of the agents on the expression of the transcription factors and of insulin. The expression of four transcription factors important in the development of the B-cell was analysed: Pdx-1, Nk6.1, Neuro D and Neurogenin 3, as well as the expression of insulin.

### Immunocytochemistry

Because the RT-PCR technique does not permit to know whether the number of precursors cells or the number of transcripts was increased, the cells were marked with antibodies against Pdx-1, Nkx6.1 and insulin.

### Antibodies

The markers Pdx-1, Nkx6.1 and insulin were detected with anti-frog Pdx-1 cross reactive with mouse Pdx-1 (provided by A. Skoudy, IMIM, Barcelona), anti-Nkx6.1 (provided by A. Skoudy, IMIM, Barcelona ) and anti-insulin (SIGMA, I-2018).

### Immunolabeling

At the end of the differentiation period, the cells were washed several times in PBS and fixed for 10 min with 4% paraformaldehyde in PBS. After fixation, the cells were washed for 15 min with PBS again and permeabilized with 1% Triton X-100 (Sigma, Madrid) for 2 min. To reduce nonspecific antibody binding, cells were first preincubated with blocking buffer (10% serum in phosphate-bufferd saline for 15 min at room temperature) before primary antibodies were applied in the same buffer. Anti-insulin monoclonal antibody (1:1000 dilution; Sigma, Madrid), anti-Pdx-1 or anti-Nkx6.1 were applied overnight at 4°. After washing, rhodamine-conjugated secondary antibodies (1:300 dilution) were applied for 2 h to visualize staining of primary antibodies. Location of staining was always intracellular. Cells stained were visualized using confocal microscopy with adequate filters to rhodamine. Optical sections was > 8µm and the optical slice of 2 µm. Total number of cells in the culture was obtained by nuclear staining with DAPI (D-1306 Molecular probes).

Table I represents the proportion of cells that were positive for different markers after some of the treatments described before. Total number of cells was determined by nuclear staining with DAPI. Incubation with pancreases taken from mouse embryonic day 17.5 was very effective increasing insulin, Pdx-1 and Nkx6.1. Particularly important are the results of double staining with insulin and Pdx-1 antibodies which reaches percentages close to 20% of the total number of cells. Whilst the presence of a protein is necessary, but not sufficient, to identify a cell type, the simultaneous expression of Pdx-1 and insulin is unique for the pancreatic B-cell.

**TABLE I:**

| **Effect of differentiation factors on the percentage of cells positive for islet cell markers.** Effects of pancreasses and anti-Sonic hedgehog (Anti-Shh, 10µg/ml) were assayed applying them during the embryoid body phase (EB) (7 days). Nicotinamide (10 mM) and LY294002 (20 µM) were applied during the embryoid body phase (7 days) plus the plating period (7 days) (EB7 + P7). ES: undifferentiated cells. | | | | |
|---|---|---|---|---|
| | **Insulin** | **Pdx1** | **Nkx6.1** | **Pdx1+ Insulin** |
| **ES** | <1%(14) | <1%(12) | < 1% (11) | <1%(7) |
| **EB7 + P7** | 1.1 ± 0.8 % (3) | 5.5 ± 2.5 % (6) | 8.0 ± 1.3% (3) | < 1 % (3) |
| **Pancreases** | 18.5 ± 3.0 % (8) | 32.5 ± 2.5 % (8) | 16.1 ± 5.4 % (4) | 18.3 ± 1.7 % (4) |
| **Anti-Shh** | 4.5 ± 1.5 % (8) | 22.5 ± 3.6 % (6) | 9.7 ± 3.7 % (3) | 4.7 ± 1.4 % (3) |
| **Nicotinamide** | 7.2 ± 4.1 % (11) | 14.3 ± 3.8 % (5) | 20.0 ± 1.5 % (3) | 9.4 ± 3.5 % (3) |
| **LY294002** | 6.5 ± 3.0 % (11) | 11.7 ± 3.5 % (5) | 7.8 ± 3.1 % (3) | 8.2 ± 2.6 % (3) |

### Use of a cell trapping system

### Generation of the construction used for transfection

A DNA molecule containing the human insulin/βgeo gene and a phosphoglycerate kinase-hygromycin resistant gene (pGK-hygro) in a pBSII-SK (Stratagene, La Jolla, CA, USA) common vector was constructed. Human insulin gene was double digested with BssSI (New England Biolabs, IZASA SA, Barcelona, Spain) and SexA1 (Roche Diagnostics, Barcelona, Spain) at the level of exon 2, and the βgeo fragment was inserted (Friedrich G, Soriano P (1991) Genes Dev 5: 1513-1523). This construction was then digested at the 3' end of the insulin gene with *Xho*I and blunted to insert the pGK-hygro (*Bgl*II-blunt). In both cases, right orientation was verified after ligation. Plasmid was linearized by *Xbal* digestion and transfected in R1 ES cells by electroporation. Transfected clones are selected by growth in the presence of 200 µg/ml hygromycin (Calbiochem, La Jolla, CA, USA). Transfected ES cells were cultured in high-glucose Dulbecco's modified Eagle's medium (DMEM) (GIBCO/BRL, Grand Island, NY, USA) containing 10 % fetal bovine serum (FBS) (GIBCO/BRL), 1 % nonessential amino acids (GIBCO/BRL), 0.1 mmol/1 2-mercaptoethanol (GIBCO/BRL), 1 mmol/l sodium pyruvate, 100 IU/ml penicillin, and 0.1 mg/ml streptomycin. The undifferentiated state was maintained by 1,000 U/ml recombinant leukemia inhibitory factor (LIF) (GIBCO/BRL) as previously described (Smith AG (1991) J. Tissue Culture Methods 13: 89-94).

### In vitro differentiation

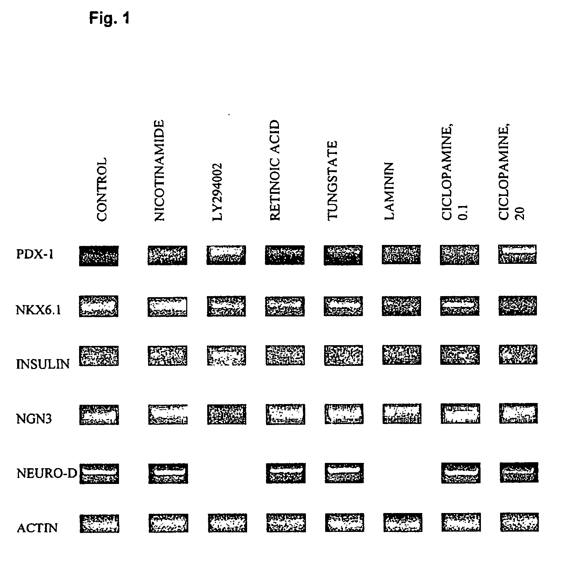

To induce differentiation to an insulin-secreting cell line, 2 x 10⁶ hygromycin-resistand ES cells were plated onto a 100-mm bacterial Petri dish and cultured in DMEM lacking supplemental LIF. Additionally one factor selected from the group consisting of mice pancreasses, nicotinamide (10 mM), LY294002 (15-20 µM), cyclopamine (100 nM-20 µM), anti-Sonic Hedgehog (10 µg/ml), laminin-1 (40 µg/ml), basic fibroblast growth factor (5-10 ng/ml), sodium tungstate (1 µM), all-trans retinoic acid (1 µM), was added. After 5-10 days in suspension culture,the resulting embryoid bodies were mechanically disaggregated and then plated onto gelatin-coated 100 mm tissue culture dishes during 7-14 additional days. Additionally, at least one factor from the group formed by nicotinamide (10 mM), LY294002 (15-20 µM), cyclopamine (100 nM-20 µM), anti-Sonic Hedgehog (10 µg/ml), laminin-1 (40 µg/ml), basic fibroblast growth factor (5-10 ng/ml), sodium tungstate (1 µM), all-trans retinoic acid (1 µM), was added. This procedure resulted in cultures in which the proportion of Pdx1-positive cells, insulin-positive cells and Nkx6.1-positive cells was substantially increased (see Table I). Gene expression of islet markers, as determined by RT-PCR also showed an increase in the expression of relevant markers (Figures 1, 2 and 3).

For ES Ins/βgeo selection, the differentiated cultures were grown in the same medium in the presence of 200 µg/ml G418 (GIBCO/BRL). For final differentiation and maturation, the resulting clones were trypsinized and plated in a 100-mm bacterial Petri dish and grown for 14 days in DMEM supplemented with 200 µg/ml G418 and 10 mmol/l nicotinamide (Sigma, Madrid). Finally the resulting clusters were cultured for 1-5 days in RPMI 1640 supplemented with 10 % FBS, 10 mmol/l nicotinamide, 200 µg/ml G418, 100 IU/ml penicillin, 0.1 mg/ml streptomycin, and 5.6 mmol/l glucose.

### Figures

Figure 1 shows the results of the reverse transcription-polymerase chain reaction after agarose gel electrophoresis of the PCR products. The effect of exogenous factors on the expression of genes which characterise islet cells and/or islet precursors can be seen (Insulin, Pdx1, Nkx6.1, Ngn3 and Beta2/NeuroD). β-Actin transcript amplification is shown as a control of RNA amount and integrity. Control band: gene expression in undifferentiated ES D3 cells. Nicotinamide (10 mM), LY294002 (20 µM), cyclopamine (100 nM and 20 µM), laminin-1 (40 µg/ml), sodium tungstate (1 µM), all-trans retinoic acid (1 µM), were added both during 7 days in the embryoid body phase and 7 days more during subsequent plating.

Nicotinamide (10 mM) and LY 294002 (20 µM) were the most effective promoting the expression of insulin, Pdx1 and Nkx6.1, although the latter seems to be more potent promoting Pdx1 whilst decreasing Neurogenin3 expression. Increase in Pdx1 expression reflects an increase in islet precursors (Jonsson J, Carlsson L, Edlund T, Edlund H (1994) Nature 371: 606-609; Offield MF, Jetton TL, Labosky PA, Ray M, Stein RW, Magnuson MA, Hogan BL, Wright CV (1996) Development 122: 983-995; Ohlson H, Karlsson K, Edlund T.(1993) EMBO J 12: 4251-4259; Wang H, Maechler P, Ritz-Laser B, Hagenfeldt KA, Ishihara H, Philippe J, Wollheim CB (2001) J. Biol. Chem 276: 25279-25286). Retinoic acid (1 µM) and sodium tungstate (1 µM) were less effective increasing Pdx1 and insulin expression, but were potent activators of neurogenin3, considered to be an islet precursor marker (Jensen, J, Heller, RS, Funder-Nielsen, T, Pedersen, EE, Lindsell, C, Weinmaster, G, Madsen, OD, Serup, P (2000) Diabetes 49: 163-176; Schwitzgebel, VM, Scheel, DW, Conners, JR, Kalamaras, J, Lee, JE, Anderson, DJ, Sussel, L, Johnson, JD, German, MS (2000) Development 127: 3533-3542; Gradwohl G, Dierich A, LeMeur M, Guillemot F (2000) Proc NatI Acad Sci USA 97: 1607-1611). The *Veratrum* alkaloid ciclopamine, known to repress Sonic Hedgehog expression promoted Pdx1 and Neurogenin3 expression.

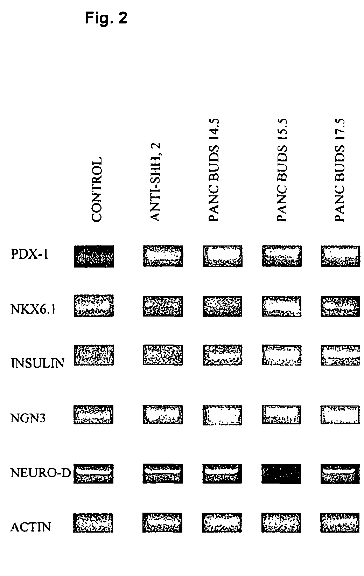

Figure 2 shows the effect of agents added during embryoid body phase formation (7 days). β-Actin transcript amplification is shown as a control of RNA amount and integrity. Control band: gene expression in undifferentiated ES D3 cells. Anti-Sonic Hedgehog (10 µg/ml) was very effective increasing the expression of Pdx1, Nkx6.1, Insulin, Neurogenin3 and Beta2/NeuroD. The most effective factor promoting ES cell differentiation was the incubation with embryonic pancreasses isolated from mouse embryonic days 14.5 to 17.5.

The representative examples of the strategies used demonstrate that the proportion of islet precursors and islet-like cells may be increased during in-vitro differentiation.

## Claims

1. Process for the production of islet beta cells or islet beta cell-like cells from omnipotent or pluripotent cells wherein the production is induced by the addition of at least one compound selected from the group consisting of embryonic pancreases and parts thereof, sonic hedgehog inhibitors, especially cyclopamin, anti-Sonic hedgehog or basic fibroblast growth factor like bFGF or FGF2, nicotinamide, LY294002, sodium tungstate, laminin-1 or another extracellular matrix protein and all-trans retinoic acid.

2. Process according to claim 1, wherein glucose is used in a low concentration.

3. Process according to claim 2, wherein glucose is used in a concentration between 1 and 25 mM.

4. Process according to claim 1, wherein the totipotent or pluripotent cells are embryonic or adult stem cells.

5. Process according to claim 4, wherein the embryonic stem cells are stem cells from mammals.

6. Process according to claim 5, wherein the embryonic stem cells from mammal are from the cell line D3 from mouse.

7. Process according to claim 1, wherein the pluripotent cells are cells of the pancreatic bud, endocrine cell precursors, endocrine cells, endodermal cells or beta-cell precursors.

8. Process according to claim 1, wherein beta-cell-like cells are cells which contain and/or produce an elevated amount of at least one islet marker.

9. Process according to claim 8, wherein the islet marker is insulin or a transcription factor selected from the group consisting of Pdx-1, Nkx6.1, Neurogenin3 and/or Beta2/Neuro-D.

10. Process according to claim 1, wherein beta-cell-like cells are beta-cell precursors, endocrine or endodermal cells.

11. Process according to claim 1, wherein for the differentiation of the cells they are plated onto a gel after the formation of the embryoid body.

12. Process according to claim 11, wherein a gelatin-containing gel is used.

13. Process according to claim 1, wherein the formation of the embryoid body occurs for a time between 4 and 12 days.

14. Process according to claim 13, wherein the formation of the embryoid body is taking place for a time of approximately 7 days.

15. Process according to claim 11, wherein the formation of the embryoid body is taking place in a medium containing fetal calf serum in a concentration between 0 and 6 per cent.

16. Process according to claim 15, wherein concentration of the fetal calf serum ist approximately 3 per cent.

17. Process according to claim 11, wherein the differentiation of the cells after formation of the embryoid body is taking place for a time between 4 and 30 days.

18. Process according to claim 17, wherein the differentiation of the cells after formation of the embryoid body is taking place for a time of approximately 7 or approximately 14 days.

19. Process according to claim 1, wherein the omnipotent or pluripotent cells are transfected with a marker which is enabling selection of a cell lineage which is able to differentiate to beta-cells or beta-cell-like cells and where this marker is used for selection or enrichment of such cell lineages which are able to differentiate to beta-cells.

20. Process according to claim 19, wherein the marker additionally allows selection or enrichment of transfected cells towards a background of non-transfected cells.

21. Islet beta cells or beta cell-like cells, obtainable by a process according to claim 1.

22. Organ or tissue, containing cells according to claim 21.

23. Process for the preparation of an organ or tissue according to claim 22, wherein cells according to claim 21 are implanted into the organ or tissue.

24. A drug product comprising at least one cell according to claim 21 and, if appropriate, suitable additives and/or adjuvants.

25. A process for the preparation of a a drug product according to claim 24, where at least one cell according to claim 21 is formulated together with a pharmaceutically acceptable additive and/or adjuvant.

26. Use of a drug product according to claim 24 for the treatment of diabetes type 1.

27. A diagnostic agent comprising a cell according to claim 21 and, if appropriate, suitable additives and/or adjuvants.

28. A process for the preparation of a a diagnostic agent according to claim 27, where at least one cell according to claim 21 is formulated together with a pharmaceutically acceptable additive and/or adjuvant.

## Amended claims

### Amended claims in accordance with Rule 86(2) EPC.

**1.** Process for the production of islet beta cells or islet beta cell-like cells from stem cells wherein the stem cells are treated with at least one compound selected from the group consisting of pancreases and parts thereof and exogenous factors.

**2.** Process according to claim 1, wherein exogenous factors are selected from the group consisting of sonic hedgehog inhibitors, especially cyclopamine, anti-Sonic hedgehog or basic growth factor like bFGF or FGF2, nicotinamide, LY294002, sodium tungstate, laminin-1 or another extracellular matrix protein and all-trans retinoic acid.

**3.** Process according to claim 1, wherein the stem cells are adult stem cells.

**4.** Process according to claim 1, wherein the stem cells are embryonic germ cells.

**5.** Process according to claim 1, wherein the stem cells are embryonic stem cells.

**6.** Process according to claim 5, wherein the embryonic stem cells are stem cells from mammals.

**7.** Process according to claim 1, wherein the stem cells are embryonic carcinoma cells.

**8.** Process according to claim 4 or 5, wherein the exogenous factors and/or pancreases are added at least during the formation of the embryoid body.

**9.** Process according to claim 8, wherein the formation of the embryoid body occurs for a time between 4 and 12 days.

**10.** Process according to claim 9, wherein the formation of the embryoid body is taking place for a time of approximately 7 days.

**11.** Process according to claim 8, wherein the formation of the embryoid body is taking place in a medium containing fetal calf serum in a concentration between 0 and 6 per cent.

**12.** Process according to claim 11, wherein concentration of the fetal calf serum is approximately 3 per cent.

**13.** Process according to claim 1 wherein the factors are added, when the factor inhibiting cell differentiation is left out from the medium.

**14.** Process according to claim 4 or 5, wherein for further differentiation the cells are plated onto a gel after the formation of the embryoid body.

**15.** Process according to claim 14, wherein a gelatin-containing gel is used.

**16.** Process according to claim 14 or 15, wherein the exogenous factors are added continuosly at periodic intervals, also after the embryoid body has been plated on the culture plates.

**17.** Process according to claim 16, wherein the exogenous factors are selected from the group consisting of nicotinamide, LY294002, sodium tungstate, retinoic acid and cyclopamine.

**18.** Process according to claim 4 or 5, wherein the differentiation of the cells after formation of the embryoid body is taking place for a time between 4 and 20 days.

**19.** Process according to claim 18, wherein the differentiation of the cells after formation of the embryoid body is taking place for a time of approximately 7 or approximately 14 days.

**20.** Process according to claim 8, wherein addition of nicotinamide, LY294003, sodium tungstate, retinoic acid and/or cyclopamine takes place continuosly at periodic time intervals, also after the formation of the embryoid body.

**21.** Process according to claim 8, wherein the exogenous factors and/or pancreases are added only during the formation of the embryoid body.

**22.** Process according to claim 21, wherein the exogenous factors are anti-Shh and/or laminin-1.

**23.** Process for the production of islet beta cells or islet beta cell-like cells from omnipotent or pluripotent cells wherein the omnipotent or pluripotent cells are treated with at least one compound selected from the group consisting of pancreases and parts thereof.

**24.** Process according to claim 1 or claim 23, wherein glucose is used in a low concentration.

**25.** Process according to claim 24, wherein glucose is used in a concentration between 1 and 25 mM.

**26.** Process according to claim 1 or 23, wherein beta-cell-like cells are cells which contain and/or produce an elevated amount of at least one islet marker.

**27.** Process according to claim 26, wherein the islet marker is insulin or a transcription factor selected from the group consisting of Pdx-1, Nkx6.1, Neurogenin3 and/or Beta2/Neuro-D.

**28.** Process according to claim 1 or 23, wherein beta-cell-like cells are beta-cell precursors, endocrine or endodermal cells.

**29.** Process according to claim 1, wherein the stem cells are transfected with a marker which is enabling selection of a cell lineage which is able to differentiate to beta-cells or beta-cell-like cells and where this marker is used for selection or enrichment of such cell lineages which are able to differentiate to beta-cells.

**30.** Process according to claim 23, wherein the omnipotent or pluripotent cells are transfected with a marker which is enabling selection of a cell lineage which is able to differentiate to beta-cells or beta-cell-like cells and where this marker is used for selection or enrichment of such cell lineages which are able to differentiate to beta-cells.

**31.** Process according to claim 29 or 30, wherein the marker additionally allows selection or enrichment of transfected cells towards a background of non-transfected cells.

**32.** Islet beta cells or beta cell-like cells, obtainable by a process according to claim 1 or 23.

**33.** Organ or tissue, containing cells according to claim 32.

**34.** Process for the preparation of an organ or tissue according to claim 33, wherein cells according to claim 32 are implanted into the organ or tissue.

**35.** A drug product comprising at least one cell according to claim 32 and, if appropriate, suitable additives and/or adjuvants.

**36.** A process for the preparation of a a drug product according to claim 35, where at least one cell according to claim 32 is formulated together with a pharmaceutically acceptable additive and/or adjuvant.

**37.** Use of a drug product according to claim 35 for the treatment of diabetes type 1.

**38.** A diagnostic agent comprising a cell according to claim 32 and, if appropriate, suitable additives and/or adjuvants.

**39.** A process for the preparation of a a diagnostic agent according to claim 38, where at least one cell according to claim 32 is formulated together with a pharmaceutically acceptable additive and/or adjuvant.
